# EUROPEAN PATENT APPLICATION

(11) **EP 4 289 443 A2**
(43) Date of publication of application: **13.12.2023**
(21) Application number: 23196633.4
(22) Date of filing: 23.02.2018
(51) Int. Cl.: A61K 39/395

(54) **STABILIZED ANTIBODY PROTEIN SOLUTIONS**

(30) Priority: 24.02.2017 GB 201703063
(62) Divisional of application: 18708465.2
(71) Applicant: Arecor Limited, Little Chesterford Saffron Walden CB10 1XL (GB)
(72) Inventor: JEZEK, Jan, Saffron Walden, CB10 1XL (GB); GERRING, David, Saffron Walden, CB10 1XL (GB)
(74) Representative: Sagittarius IP

(57) **Abstract**

There is provided, *inter alia,* an aqueous solution comprising (i) an antibody protein; and (ii) a stabilizing mixture of arginine, methionine and a C3 polyol.

## Description

### BACKGROUND OF THE INVENTION

When formulated as aqueous solutions, antibody proteins are susceptible to structural degradation during storage. The processes involved in protein degradation can be divided into physical (e.g. loss of quaternary, tertiary or secondary structure, aggregation, particle formation) and chemical (i.e. processes involving a covalent change such as deamidation, aspartate isomerization, oxidation, hydrolytic clipping etc.). Each of the degradants (e.g. soluble aggregated species, insoluble aggregated species and chemically modified variants) can impact the biological activity, toxicity or immunogenicity of the antibody protein.

Therefore, the level of all degradants has to be kept within the tight specifications that are set for each antibody protein product. The rates of the degradation processes depend on temperature and antibody proteins are generally more stable at lower temperatures. Consequently, commercial antibody products must typically be stored refrigerated. However, with increasing trend toward subcutaneous products that can be self-administered by the patient, there is a strong need to develop antibody protein products that can be used outside the cold chain, at least for a period of time, such as 2 weeks, such as 4 weeks, such as 12 weeks or more. The ability to store the product outside the cold chain often results in considerable improvement in convenience for the patient during the in-use period. Allowed excursions outside the cold chain can also significantly improve shipment logistics.

The present invention addresses the problem of instability of antibody proteins, in particular the problem of antibody protein degradation.

WO2006/081587A2 (Wyeth) describes formulations for maintaining the stability of polypeptides, in particular therapeutic antigen-binding polypeptides such as antibodies. The formulations generally include an antioxidant in a sufficient amount as to inhibit by-product formation, for example, the formation of high molecular weight polypeptide aggregates, low molecular weight polypeptide degradation fragments, and mixtures thereof. The formulations described optionally comprise a tonicity agent, such as mannitol, and a buffering agent or amino acid such as histidine.

WO2007/109221A2 (Wyeth) describes methods of reducing aggregation of a protein in a formulation, comprising adding methionine to the formulation to a concentration of about 0.5 mM to about 145 mM, wherein the method results in reduced aggregation of the protein in the formulation compared with the protein in a formulation lacking methionine.

EP2238985A1 (Chugai Seiyaku) sets out to provide an antibody-containing formulation which is stable and suited for subcutaneous administration, wherein dimerization is prevented during long-term storage. An antibody-containing liquid formulation containing arginine and methionine is described.

WO03/072060A2 (Immunex Corporation) describes a stable aqueous pharmaceutical formulation comprising a therapeutically effective amount of an Fc domain containing polypeptide, and an aggregation inhibitor selected from the group consisting of L-arginine and L-cysteine.

### SUMMARY OF THE INVENTION

The present invention addresses the problem of instability of antibody proteins. In one embodiment, the invention relates to an aqueous solution comprising (i) an antibody protein; and (ii) a stabilizing mixture of arginine, methionine and a C3 polyol. In one embodiment, the invention provides a method of stabilizing an antibody protein in an aqueous solution to storage comprising the step of adding to the solution a mixture of arginine, methionine and a C3 polyol.

### DETAILED DESCRIPTION OF THE INVENTION

The present invention relates to the discovery that an aqueous solution of antibody protein can be stabilized by a mixture of arginine, methionine and a C3 polyol.

The term "aqueous solution", as used herein, refers to a solution in water, preferably distilled water, deionized water, water for injection, sterile water for injection or bacteriostatic water for injection. The aqueous solutions of the invention include dissolved antibody protein, arginine, methionine and a C3 polyol, and optionally, one or more additives and/or excipients. The aqueous solutions can also include one or more components, such as additives or excipients, which are partially dissolved or undissolved. The presence of such component or components will result in a multi-phase composition, such as a suspension or an emulsion. Preferably, the aqueous solution of the invention is a homogeneous solution, as determined by eye or by light-scattering.

The term "antibody protein", as used herein refers to an antibody, an antibody fragment, an antibody conjugated to an active moiety, a fusion protein comprising one or more antibody fragments, such as an immunoglobulin Fc domain, or a derivative of any of the aforementioned. Examples of derivatives include conjugated derivatives e.g. an antibody or antibody fragment conjugated to another moiety. Such moieties include chemically inert polymers such as PEG. Preferred antibodies include monoclonal antibodies and polyclonal antibodies, preferably monoclonal antibodies. The monoclonal antibodies can be, for example, mammalian (e.g. murine) or avian, chimeric, for example, human/mouse or human/primate chimeras, humanized antibodies or fully human antibodies. Suitable antibodies include an immunoglobulin, such as IgG, including IgG₁, IgG₂, IgG₃ or IgG₄, IgM, IgA, such as IgAi or IgA₂, IgD, IgE or IgY. Suitable antibodies also include single chain antibodies. Also included are antibody fragments including Fc, Fab, Fab₂, ScFv fragments and the like. Also embraced are single domain antibodies including Nanobodies.

In certain embodiments, the antibody is fused or conjugated to an active molecule, such as a toxin or a chelating agent capable of binding a radioactive metal ion, such as ⁹⁹Tc, ¹¹¹Ir, ¹³¹I or ⁹⁰Y. In such embodiments, the antibody typically functions as a targeting agent, for example, directing the active molecule to cells which display a certain cell surface protein.

Specific antibodies which can be formulated as described herein include, but are not limited to, infliximab (chimeric antibody, anti-TNFα), basiliximab (chimeric antibody, anti-IL-2), abciximab (chimeric antibody, anti- GpIIb/IIIa), daclizumab (humanized antibody, anti-IL-2), gemtuzumab (humanized antibody, anti-CD33), alemtuzumab (humanized antibody, anti-CD52), edrecolomab (murine Ig2a, anti-EpCAM), rituximab (chimeric antibody, anti-CD20), palivizumab (humanized antibody, anti-respiratory syncytial virus), trastuzumab (humanized antibody, anti- HER2/neu(erbB2) receptor), bevacizumab (humanized antibody, anti-VEGF), cetuximab (chimeric antibody, anti-EGFR), eculizumab (humanized antibody, anti-complement system protein C5), efalizumab (humanized antibody, anti-CD 1Ia), ibritumomab (murine antibody, anti-CD20), muromonab-CD3 (murine antibody, anti- T cell CD3 receptor), natalizumab (humanized antibody, anti-α4 integrin), nimotuzumab (humanized IgGl, anti-EGF receptor), omalizumab (humanized antibody, anti-IgE), panitumumab (human antibody, anti-EGFR), ranibizumab (humanized antibody, anti-VEGF), 1-131 tositumomab (humanized antibody, anti-CD20), ofatumumab (human antibody, anti-CD-20), certolizumab (humanized antibody, anti-TNF-α), golimumab (human antibody, anti-TNFα) and denosumab (human antibody, anti-RANK ligand). Preferred antibodies include trastuzumab, rituximab, bevacizumab, cetuximab and ipilimumab. In one embodiment, the monoclonal antibody is bevacizumab. In another embodiment, the monoclonal antibody is rituximab.

Other chimeric antibodies which can be formulated as described herein include bavituximab (anti-phosphatidylserine), brentuximab (anti-CD30), siltuximab (anti-IL-6), clenoliximab (anti-CD4), galiximab (anti-CD80), gomiliximab (anti-CD23), keliximab (anti-CD4), lumiliximab (anti-CD23), priliximab (anti-CD4), teneliximab (anti-CD40), vapaliximab (anti-VAP1), ecromeximab (anti-GD3), and pagibaximab (anti-staphylococcal lipoteichoic acid).

Other humanized antibodies which can formulated as described herein include epratuzumab (anti-CD22), afutuzumab (anti-CD20), bivatuzumab mertansine (anti-CD44), cantuzumab mertansine (anti-mucin), citatuzumab bogatox (anti-TACSTD1), dacetuzumab (anti-CD40), elotuzumab (anti-CD319), etaracizumab (anti-αᵥβ₃-integrin), farletuzumab (anti-FRα), inotuzumab ozogamicin (anti-CD22), labetuzumab (anti-carcinoembryonic antigen), lintuzumab (anti-CD33), milatuzumab (anti-CD74), nimotuzumab (anti-EGFR), oportuzumab monatox (anti-EpCAM), pertuzumab (anti-HER2), sibrotuzumab (anti-FAP), tacatuzumab tetraxetan (anti-alpha-fetoprotein), tigatuzumab (anti-TRAIL-2), tucotuzumab celmoleukin (anti-EpCAM), veltuzumab (anti-CD20), aselizumab (anti-CD62L), apolizumab (anti-HLA-DRB), benralizumab (anti-CD125), cedelizumab (anti-CD4), epratuzumab (anti-CD22), erlizumab (anti-CD18), fontolizumab (anti-interferon-γ), mepolizumab (anti-IL5), ocrelizumab (anti-CD20), pascolizumab (anti-IL4), pexelizumab (anti-complement component 5), PRO-140 (anti-CCR5), reslizumab (anti-IL5), rontalizumab (anti interferon-α), rovelizumab (anti-CD11, CD18), siplizumab (anti-CD2), talizumab (anti-IgE), teplizumab (anti-CD3), tocilizumab (anti-IL6R), vedolizumab (anti-α₄β₇-integrin), visilizumab (anti-CD3), ibalizumab (anti-CD4), tefibazumab (anti-clumping factor A), tadocizumab (anti-α_{11b}β₃-integrin), bapineuzumab (anti-amyloid-β), solanezumab (anti-amyloid-β), tanezumab (anti-NGF), urtoxazumab (anti-E. coli Shiga-like toxin II B subunit), felvizumab (anti-respiratory syncytial virus), motavizumab (anti- respiratory syncytial virus glycoprotein F) and lebrikizumab (anti-IL13).

Additional human antibodies which can be formulated as described herein include atorolimumab (anti-Rh factor), fresolimumab (anti-TGFβ-1, -2, and -3), lerdelimumab (anti-TGFβ-2), metelimumab (anti-TGFβ-1), morolimumab (anti-Rh factor), ipilimumab (anti-CTLA-4), tremelimumab (anti-CTLA-4), bertilimumab (anti-CCL11), zanolimumab (anti-CD4), briakinumab (anti-IL12, -23), canakinumab (anti-IL1β), ustekinumab (anti-IL12, -23), adecatumumab (anti-EpCAM), belimumab (anti-B cell activating factor), cixutumumab anti-IGF-1 receptor), conatumumab (anti-TRAIL-R2), figitumumab (anti-IGF-1 receptor), iratumumab (anti-CD30), lexatumumab (anti-TRAIL-R2), lucatumumab (anti-CD40), mapatumumab (anti-TRAIL-R4), necitumumab (anti-EGFR), olaratumab (anti-PDGF-Rα), pritumumab (anti-vimentin), robatumumab (anti-IGF-1 receptor), votumumab (anti-tumor antigen CTAA16.88), zalutumumab (anti-EGFR), stamulumab (anti-myostatin), efungumab (anti-fungal HSP90), exbivirumab (anti-hepatitis B surface antigen), foravirumab (anti- rabies glycoprotein), libivirumab (anti-hepatitis B surface antigen), rafivirumab (anti- rabies glycoprotein), regavirumab (anti-cytomegalovirus glycoprotein B), sevirumab (anti-cytomegalovirus), tuvirumab (anti-hepatitis B virus), panobacumab (anti-pseudomonas aeruginosa serotype IATS 011), raxibacumab (anti-anthrax toxin), ramucirumab (anti-VEGF-R2), and gantenerumab (anti-amyloid-β).

Fusion proteins comprising a fragment of an immunoglobulin molecule can also be formulated according to the invention. Suitable fusion proteins include proteins comprising an active protein domain fused to one or more immunoglobulin fragments, such as Fc domains. Such fusion proteins include dimeric proteins having monomeric units comprising an active protein domain, such as a soluble receptor or a receptor extracellular ligand binding domain, which is fused to an immunoglobulin Fc domain. Two Fc domains can associate via disulfide bonds to form the dimeric protein. Such fusion proteins include etanercept, abatacept and belatacept.

Conjugated derivatives comprising antibodies (or one or more antibody fragments) and a chemically inert polymer such as PEG can also be formulated according to the invention. Such derivatives include certolizumab pegol.

The antibody protein can be isolated from natural sources or be a recombinant protein.

In certain embodiments, the antibody protein is substantially pure, that is, the composition comprises a single antibody protein and no substantial amount of any additional protein. In preferred embodiments, the antibody protein comprises at least 99%, preferably at least 99.5% and more preferably at least about 99.9% of the total protein content of the composition. In preferred embodiments the antibody protein is sufficiently pure for use as in a pharmaceutical composition.

The antibody protein is preferably a therapeutic antibody protein. Such an antibody protein has a desirable therapeutic or prophylactic activity and is indicated for the treatment, inhibition or prevention of a disease or medical disorder.

In one embodiment, antibody protein is a monoclonal antibody such as trastuzumab, rituximab, bevacizumab, cetuximab and ipilimumab. In one embodiment, the antibody protein is a monoclonal antibody such as infliximab. In another embodiment, the antibody protein is a fusion protein comprising an active protein domain fused to one or more immunoglobulin Fc fragments such as etanercept, abatacept or belatacept. In a further embodiment, the antibody is a derivative of an antibody protein and is a conjugated derivative comprising one or more antibodies or antibody fragments and a chemically inert polymer, such as certolizumab pegol.

The antibody protein is suitably present at a concentration of about 1 mg/mL to about 300 mg/mL, such as about 10 mg/mL to about 300 mg/mL, about 1 mg/mL to about 200 mg/mL or about 10 mg/mL to about 200 mg/mL.

The aqueous solution of the present invention comprises arginine, suitably L-arginine. The arginine can be added to the aqueous solution in the form of a free base or in the form of a salt such as arginine hydrochloride. The arginine has a stabilizing effect (particularly with respect to reducing aggregation) and is typically present in the aqueous solution at a concentration of about 5 mM to about 100 mM, such as about 20 mM to about 80 mM, e.g. about 60 mM or about 80 mM.

The aqueous solution of the present invention also comprises methionine, suitably L-methionine. The methionine can be added to the aqueous solution in the form of a free base or in the form of a salt such as methionine hydrochloride. The methionine has a stabilizing effect (particularly with respect to reducing aggregation) and is typically present in the aqueous solution at a concentration of about 2 mM to about 50 mM, such as about 10 mM to about 40 mM, e.g. about 30 mM.

Typically, the pH of the aqueous solution of the present invention is between about pH 4.0 and about pH 8.0, such as between about pH 5.0 and about pH 7.0 or between about pH 5.0 and about pH 6.5.

In one embodiment the aqueous solution of the invention further comprises a buffer in order to stabilise the pH of the formulation, which can also be selected to enhance antibody protein stability. While arginine and methionine may have buffering capacity, they typically do not act as buffers at in the pH range of between about pH 4.0 and about pH 8.0, which is a suitable pH range for the present aqueous solution. Hence, the aqueous solution may further comprise a buffer which is other than arginine or methionine.

Suitably the buffer is selected from the group consisting of histidine, succinate, maleate, acetate, phosphate and TRIS. In an embodiment, the buffer is phosphate. In a further embodiment, the buffer is citrate e.g. trisodium citrate.

In one embodiment, a buffer is selected to have a pKₐ close to the pH of the composition; for example, histidine is suitably employed as a buffer when the pH of the composition is in the range 5.0-7.0. As another example, phosphate is suitably employed as a buffer when the pH of the composition is in the range 6.1-8.1. Alternatively, in another embodiment, the solution of the invention is further stabilised as disclosed in WO2008/084237A2, which describes a formulation comprising a protein and one or more additives, characterised in that the system is substantially free of a conventional buffer, i.e. a compound with an ionisable group having a pKₐ within 1 unit of the pH of the formulation at the intended temperature range of storage of the composition, such as 25 °C. In this embodiment, the pH of the formulation is set to a value at which the formulation has maximum measurable stability with respect to pH; the one or more additives (displaced buffers) are capable of exchanging protons with the insulin compound and have pKₐ values at least 1 unit more or less than the pH of the formulation at the intended temperature range of storage of the formulation. The additives may have ionisable groups having pKₐ between 1 to 5 pH units, preferably between 1 to 3 pH units, most preferably from 1.5 to 2.5 pH units, of the pH of the aqueous formulation at the intended temperature range of storage of the composition (e.g. 25 °C). Such additives may typically be employed at a concentration of 0.5-10 mM e.g. 2-5 mM.

Typically, the buffer is present at a concentration of about 0.5 mM to about 50 mM, such as about 1 mM to about 20 mM, e.g. about 2 mM to about 5 mM.

The aqueous solutions of the invention may optionally comprise a surfactant. In one embodiment, the surfactant is a non-ionic surfactant such as an alkyl glycoside e.g. dodecyl maltoside; a polysorbate surfactant such as polysorbate 80 or polysorbate 20; an alkyl ether of polyethylene glycol e.g. selected from polyethylene glycol (2) dodecyl ether, polyethylene glycol (2) oleyl ether and polyethylene glycol (2) hexadecyl ether; a block copolymer of polyethylene glycol and polypropylene glycol, such as poloxamer 188, poloxamer 407, poloxamer 171 or poloxamer 185; or an alkylphenyl ether of polyethylene glycol, such as 4-(1,1,3,3-tetramethylbutyl)phenyl-polyethylene glycol. Suitably the non-ionic surfactant is present at a concentration of about 10 µg/mL to about 2000 µg/mL, such as about 50 µg/mL to about 1000 µg/mL, e.g. about 100 µg/mL to about 500 µg/mL.

The aqueous solution of the invention may cover a wide range of osmolarity, including hypotonic, isotonic and hypertonic aqueous solutions. Suitably, the aqueous solution of the invention is substantially isotonic. In one embodiment, the aqueous solution of the invention is isotonic. Suitably, the osmolarity of the aqueous solution is selected to minimize pain according to the route of administration e.g. upon injection. Preferred aqueous solutions have an osmolarity in the range of about 200 mOsm/L to about 500 mOsm/L. Preferably, the osmolarity is in the range of about 250 mOsm/L to about 350 mOsm/L. More preferably, the osmolarity is about 300 mOsm/L.

Tonicity of the aqueous solution may be adjusted with a tonicity modifier. Tonicity modifiers may be charged or uncharged.

Examples of charged tonicity modifiers include salts such as a combination of sodium, potassium, magnesium or calcium ions, with chloride, sulfate, carbonate, sulfite, nitrate, lactate, succinate, acetate or maleate ions (especially sodium chloride or sodium sulphate, particularly sodium chloride). Amino acids such as glycine or histidine may also be used for this purpose. In one embodiment, the charged tonicity modifier is selected from the group consisting of sodium chloride, sodium sulphate, sodium acetate, sodium lactate, glycine and histidine. Arginine and methionine (both required components of the aqueous solution of the invention) may function as a charged tonicity modifier. However, reference to an aqueous solution of the invention "further" comprising a charged tonicity modifier is intended to refer to an additional, further component to be added to the solution. Thus, the aqueous solution may further comprise a charged tonicity modifier which is other than arginine or methionine. Such a charged tonicity modifier is typically present at a concentration of about 25 mM to about 500 mM, such as about 50 mM to about 250 mM, e.g. about 150 mM.

Examples of uncharged tonicity modifiers include sugars, sugar alcohols and other polyols, such as sucrose, trehalose, mannitol, raffinose, lactose, dextrose, sorbitol or lactitol, or polyethylene glycols such as PEG300 or PEG400. In one embodiment, the uncharged tonicity modifier is sucrose, trehalose, mannitol, sorbitol, PEG300 or PEG400. The C3 polyol which is a required component of the aqueous solution of the invention may function as an uncharged tonicity modifier. However, reference to an aqueous solution of the invention "further" comprising an uncharged tonicity modifier is intended to refer to an additional, further component to be added to the solution. Thus, the aqueous solution may further comprise an uncharged tonicity modifier which is other than a C3 polyol, and in particular is other than 1,2-propanediol and glycerol. Such an uncharged tonicity modifier is typically present at a concentration of about 50 mM to about 1000 mM, such as about 100 mM to about 500 mM, e.g. about 300 mM.

The aqueous solution of the invention can optionally include a preservative, suitably selected from phenol, m-cresol, chlorocresol, benzyl alcohol, propylparaben, methylparaben, benzalkonium chloride and benzethonium chloride. When present, the preservative is at a concentration of about 0.01 mM to about 100 mM. A preservative selected from phenol, m-cresol, chlorocresol, benzyl alcohol, propylparaben, methylparaben may, for example, be present at a concentration of about 10 mM to about 100 mM, such as about 20 mM to about 80 mM e.g. about 25 mM to about 50 mM. A preservative selected from benzalkonium chloride and benzethonium chloride may, for example, be present at a concentration of about 0.01 mM to about 1 mM such as about 0.05 mM to about 0.5 mM e.g. about 0.05 mM to about 0.2 mM.

The present inventors have discovered that when a C3 polyol is added to an aqueous solution comprising an antibody protein, arginine and methionine, the stability of the antibody protein is surprisingly enhanced. Without wishing to be bound by theory it is believed that the additional stabilising effect of a C3 polyol is due to optimal hydrophobic and hydrogen bond interactions at the protein surface of the small polyols leading to tighter conformation and modified interfacial tension between the protein molecules, in turn leading to lower exposure of reaction sites as well as lower probability of irreversible aggregation events.

The C3 polyol is suitably selected from 1,2-propanediol (also known as propane-1,2-diol or propylene glycol) and glycerol (also known as 1,2,3-propanetriol, glycerin or glycerine). In one embodiment, the C3 polyol is 1,2-propanediol. In another embodiment, the C3 polyol is glycerol. In a further embodiment, the C3 polyol is a mixture of 1,2-propanediol and glycerol. The C3 polyol is suitably present at a concentration of about 100 mM to about 500 mM, such as about 150 mM to about 400 mM, or about 150 mM to about 200 mM. If more than one C3 polyol is present in the aqueous solution, then the concentration refers to the total concentration of C3 polyols.

In one embodiment, the ratio (mM/mM) of arginine to methionine is about between about 1:1 and about 10:1 e.g. between about 2:1 and about 6:1.

In one embodiment, the ratio (mM/mM) of arginine to C3 polyol is between about 2:1 and 1:20 e.g. between about 1:2 and about 1:10.

In one embodiment, the ratio (mM/mM) of methionine to C3 polyol is between about 1:1 and 1:40 e.g. between about 1:4 and about 1:20.

In one embodiment, the ratio (mM/mM) of the combined concentration of arginine and methionine to C3 polyol is between about 4:1 and 1:20 e.g. between about 2:1 and about 1:10.

In one embodiment, the ratio (wt/wt) of antibody protein to arginine is between about 1:10 and about 100:1 e.g. between about 1:1 and about 40:1. In another embodiment, the ratio (wt/wt) of antibody protein to arginine is between about 1:1 and about 100:1 e.g. between about 5:1 and about 40:1.

In one embodiment, the ratio (wt/wt) of antibody protein to methionine is between about 1:5 and about 200:1 e.g. between about 2:1 and about 80:1. In another embodiment, the ratio (wt/wt) of antibody protein to methionine is between about 2:1 and about 200:1 e.g. between about 10:1 and about 80:1.

In one embodiment, the ratio (wt/wt) of antibody protein to the combined weight of arginine and methionine is between about 1:15 and about 30:1 e.g. between about 1:1 and about 25:1. In another embodiment, the ratio (wt/wt) of antibody protein to the combined weight of arginine and methionine is between about 1:2 and about 50:1 e.g. between about 2:1 and about 25:1.

In one embodiment, the ratio (wt/wt) of antibody protein to C3 polyol is between about 1:5 and about 200:1 e.g. between about 2:1 and about 50:1. In another embodiment, the ratio (wt/wt) of antibody protein to C3 polyol is between about 1:2 and about 200:1 e.g. between about 2:1 and about 50:1.

The addition of a mixture of arginine, methionine and a C3 polyol to an aqueous solution of antibody protein is expected to enhance the stability of the antibody protein, e.g. as shown in Example 1. The mixture of arginine, methionine and a C3 polyol is thus referred to as a stabilizing mixture.

The "stability" of an antibody protein or a "stabilizing mixture" typically refers to a reduction of antibody protein degradation during storage. In one embodiment, "stability"/"stabilizing" refers to physical stability e.g. loss of quaternary, tertiary or secondary structure, aggregation or particle formation. In another embodiment, "stability"/"stabilizing" refers to chemical stability e.g. processes involving a covalent change such as deamidation, aspartate isomerization, oxidation or hydrolytic clipping.

It is expected that the addition of a C3 polyol to an aqueous solution comprising an antibody protein, arginine and methionine can enhance the stability of the antibody protein and in particular reduce the rate of antibody protein aggregation, compared with the same solution lacking the C3 polyol, following storage under the same conditions for the same length of time.

The present invention thus provides a method of stabilizing an antibody protein in an aqueous solution to storage comprising the step of adding to the solution a mixture of arginine, methionine and a C3 polyol. Also provided is the use of a mixture of arginine, methionine and a C3 polyol for stabilizing an antibody protein in an aqueous solution to storage. All embodiments described hereinabove with reference to the aqueous solution of the invention apply equally to the method and use of the invention.

The method of the invention refers to "the step of adding to the solution a mixture of arginine, methionine and a C3 polyol". It should be understood that the arginine, methionine and C3 polyol can be added to the solution all together at the same time, or sequentially, and in any order (i.e. "the step" may actually include multiple steps).

Also provided is a method for inhibiting formation of high molecular weight species of an antibody protein in aqueous solution during storage, comprising the step of adding to the solution a mixture of arginine, methionine and a C3 polyol.

Also provided is a method for inhibiting formation of visible particles in a solution of an antibody protein during storage, comprising the step of adding to the solution a mixture of arginine, methionine and a C3 polyol.

Also provided is a method for inhibiting formation of related species of an antibody protein in aqueous solution during storage, comprising the step of adding to the solution a mixture of arginine, methionine and a C3 polyol.

Also provided is a method for inhibiting deamidation of an antibody protein in aqueous solution during storage, comprising the step of adding to the solution a mixture of arginine, methionine and a C3 polyol.

Also provided is a method for inhibition formation of low molecular weight degradation products in an aqueous solution of an antibody protein during storage, comprising the step of adding to the solution a mixture of arginine, methionine and a C3 polyol.

Also provided is the use of a mixture of arginine, methionine and a C3 polyol for inhibiting the formation of high molecular weight species of an antibody protein in aqueous solution during storage.

Also provided is the use of a mixture of arginine, methionine and a C3 polyol for inhibiting the formation of visible particles in a composition of an antibody protein in aqueous solution during storage.

Also provided is the use of a mixture of arginine, methionine and a C3 polyol for inhibiting formation of related species of an antibody protein in aqueous solution during storage.

Also provided is the use of a mixture of arginine, methionine and a C3 polyol for inhibiting deamidation of an antibody protein in aqueous solution during storage.

Also provided is the use of a mixture of arginine, methionine and a C3 polyol for inhibiting formation of low molecular weight degradation products in an aqueous solution of an antibody protein during storage.

The term "high molecular weight species" as used herein, refers to any component of the antibody protein content which has an apparent molecular weight at least about double the molecular weight of the parent active antibody protein. That is, high molecular weight species are multimeric aggregates of the parent antibody protein. The multimeric aggregates may comprise the parent antibody protein molecules with considerably altered conformation or they may be an assembly of the parent protein units in the native or near-native conformation. The determination of high molecular weight species can be done using methods known in the art, including size exclusion chromatography, electrophoresis, analytical ultracentrifugation/sedimentation velocity, light scattering, dynamic light scattering, static light scattering and field flow fractionation.

The term "low molecular weight degradation products" as used herein, refers to any component of the antibody protein content which has an apparent molecular weight less than the molecular weight of the parent active antibody protein. That is, low molecular weight degradation products are fragments of the parent antibody protein. The determination of high molecular weight species can be done using methods known in the art, including size exclusion chromatography, electrophoresis, analytical ultracentrifugation/sedimentation velocity, light scattering, dynamic light scattering, static light scattering and field flow fractionation.

The term "related species" as used herein, refers to any component of the antibody protein content formed by a chemical modification of the parent antibody protein, such as deamidated species or oxidised species. Related species are suitably detected by cation-exchange chromatography, reversed-phase chromatography or capillary electrophoresis.

Suitably an aqueous solution of the invention is sufficiently stable such that it remains substantially free of visible particles after storage at 30 °C for at least one, two or three months. Visible particles are suitably detected using the 2.9.20. European Pharmacepoeia Monograph (Particulate Contamination: Visible Particles).

Suitably the aqueous solution of the invention is sufficiently stable such that the concentration of related species remains low upon extended storage.

In one embodiment, the aqueous solution of the invention retains at least 95%, e.g. at least 96%, e.g. at least 97%, e.g. at least 98%, e.g. at least 99% parent antibody protein (by weight of total antibody protein) after storage at 30°C for one, two or three months. The percentage of antibody protein (by weight of total antibody protein) may be determined by size-exclusion chromatography, cation-exchange chromatography, reversed-phase chromatography or capillary electrophoresis.

In one embodiment, the presence of a mixture of arginine, methionine and a C3 polyol limits the increase in high molecular weight antibody protein species to no more than 5% (by weight of total antibody protein) after storage at 40°C for one month, suitably to no more than 3% and more suitably to no more than 2%. In one embodiment, the presence of a mixture of arginine, methionine and a C3 polyol limits the increase in high molecular weight antibody protein species to no more than 5% (by weight of total antibody protein) after storage at 2-8°C for up to two years, suitably to no more than 3% and more suitably to no more than 2%. Quantitation of high molecular weight species is as percent by weight of the total antibody protein in the aqueous solution.

In one embodiment, the presence of a mixture of arginine, methionine, and a C3 polyol limits the increase in high molecular weight antibody protein species by at least 10%, preferably by at least 25%, and more preferably by at least 50% compared with an aqueous solution lacking the mixture of arginine, methionine, and a C3 polyol but otherwise identical, following storage under the same conditions and length of time.

In one embodiment, the presence of a mixture of arginine, methionine and C3 polyol maintains an aqueous solution of an antibody protein free of visible aggregates while formation of visible aggregates is observed in an aqueous solution lacking the mixture of arginine, methionine and C3 polyol but otherwise identical, following storage under the same conditions and for the same length of time. Quantification of visible aggregates can be performed by turbidity or other types of light scattering measurement.

Suitably, the aqueous solution of the invention comprises no more than 5% (by weight of total protein) high molecular weight species after storage at 40°C for at least one, two or three months. In one embodiment, the amount of high molecular weight species increases by no more than 5% (by weight of total antibody protein), preferably no more than 3%, after storage at 40°C for at least one, two or three months. Quantitation of high molecular weight species is as percent by weight of the total antibody protein in the aqueous solution.

Suitably, the aqueous solution of the invention should exhibit an increase in high molecular weight species during storage which is at least 10% lower, preferably at least 25% lower, more preferably at least 50% lower, than an aqueous solution lacking the mixture of arginine, methionine and C3 polyol but otherwise identical, following storage under the same conditions and length of time.

In one embodiment, the aqueous solution of the invention is a pharmaceutical composition suitable for administration of a therapeutic antibody protein to a subject in need thereof. Such compositions can be used in a method for administering the therapeutic protein to the subject.

In another embodiment, the invention provides a method for administering a therapeutic antibody protein to a subject in need thereof. The method comprises the step of administering an aqueous solution comprising the antibody protein, arginine, methionine and a C3 polyol. Preferably the composition is administered by intravenous, subcutaneous or intramuscular injection, or infusion. More preferably the composition is administered by subcutaneous injection.

In another embodiment, the invention provides a packaged pharmaceutical composition suitable for administration to a subject in need thereof. The pharmaceutical composition comprises an aqueous solution comprising an antibody protein, arginine, methionine and a C3 polyol. The pharmaceutical composition is preferably packaged in a vial suitable for introduction of a needle for removal of the solution. In one embodiment, the pharmaceutical composition is packaged in a glass vial with a rubber stopper. The packaged pharmaceutical composition can be provided as a kit, further comprising instructions for use and, optionally, a syringe suitable for intramuscular or subcutaneous administration. Alternatively, the packaged pharmaceutical composition can be provided in the form of a pre-filled disposable syringe suitable for intramuscular or subcutaneous administration. A pre-filled auto-injector device would also be suitable for intramuscular or subcutaneous administration.

The term "pharmaceutically acceptable", as used herein, refers to components of a pharmaceutical composition which are suitable for the intended use and mode of administration to the body of a human or an animal, such as a mammal, without undue adverse consequences, such as toxicity, irritation, and allergic response and with a reasonable risk/benefit ratio.

### Embodiments of the Invention

Embodiment 1. An aqueous solution comprising (i) an antibody protein; and
(ii) a stabilizing mixture of arginine, methionine and a C3 polyol.

Embodiment 2. A method of stabilizing an antibody protein in an aqueous solution comprising the step of adding to the solution a mixture of arginine, methionine and a C3 polyol.

Embodiment 3. Use of a mixture of arginine, methionine and a C3 polyol for stabilizing an antibody protein in an aqueous solution to storage.

Embodiment 4. The aqueous solution of embodiment 1, method of embodiment 2, or use of embodiment 3, wherein the C3 polyol is 1,2-propanediol.

Embodiment 5. The aqueous solution of embodiment 1, method of embodiment 2, or use of embodiment 3, wherein the C3 polyol is glycerol.

Embodiment 6. The aqueous solution of embodiment 1, method of embodiment 2, or use of embodiment 3, wherein the C3 polyol is a mixture of 1,2-propanediol and glycerol.

Embodiment 7. The aqueous solution, method or use of any one of embodiments 1 to 6, wherein the C3 polyol is present at a concentration of about 100 mM to about 500 mM, such as about 150 mM to about 400 mM, or about 150 mM to about 200 mM.

Embodiment 8. The aqueous solution, method or use of any one of embodiments 1 to 7, wherein the antibody protein is a therapeutic antibody protein.

Embodiment 9. The aqueous solution, method or use of any one of embodiments 1 to 8, wherein the antibody protein is an antibody, an antibody fragment, an antibody conjugated to an active moiety, a fusion protein comprising one or more antibody fragments, or a derivative of any of the aforementioned.

Embodiment 10. The aqueous solution, method or use of embodiment 9, wherein the antibody protein is a monoclonal antibody.

Embodiment 11. The aqueous solution, method or use of embodiment 10, wherein the monoclonal antibody is a murine antibody, a chimeric antibody, a humanized antibody or a human antibody.

Embodiment 12. The aqueous solution, method or use of embodiment 11, wherein the monoclonal antibody is selected from trastuzumab, rituximab, bevacizumab, cetuximab and ipilimumab.

Embodiment 13. The aqueous solution, method or use of embodiment 12, wherein the monoclonal antibody is rituximab.

Embodiment 14. The aqueous solution, method or use of embodiment 9, wherein the antibody protein is a fusion protein comprising an active protein domain fused to one or more immunoglobulin Fc fragments.

Embodiment 15. The aqueous solution, method or use of embodiment 14, wherein the antibody protein is etanercept, abatacept or belatacept.

Embodiment 16. The aqueous solution, method or use of embodiment 9, wherein the derivative is a conjugated derivative comprising one or more antibodies or antibody fragments and a chemically inert polymer.

Embodiment 17. The aqueous solution, method or use of embodiment 16, wherein the conjugated derivative is a certolizumab pegol.

Embodiment 18. The aqueous solution, method or use of any one of embodiments 1 to 17, wherein the antibody protein is present at a concentration of about 1 mg/mL to about 300 mg/mL, such as about 10 mg/mL to about 300 mg/mL, about 1 mg/mL to about 200 mg/mL or about 10 mg/mL to about 200 mg/mL.

Embodiment 19. The aqueous solution, method or use of any one of embodiments 1 to 18, wherein the arginine is present at a concentration of about 5 mM to about 100 mM, such as about 20 mM to about 80 mM, e.g. about 60 mM.

Embodiment 20. The aqueous solution, method or use of any one of embodiments 1 to 19, wherein the methionine is present at a concentration of about 2 mM to about 50 mM, such as about 10 mM to about 40 mM, e.g. about 30 mM.

Embodiment 21. The aqueous solution, method or use of any one of embodiments 1 to 20, wherein the pH of the solution is between about pH 4.0 and about pH 8.0, such as between about pH 5.0 and about pH 7.0 or between about pH 5.0 and about pH 6.5.

Embodiment 22. The aqueous solution, method or use of any one of embodiments 1 to 21, further comprising a buffer.

Embodiment 23. The aqueous solution, method or use of embodiment 22, wherein the buffer is selected from the group consisting of histidine, succinate, maleate, acetate, phosphate and TRIS.

Embodiment 24. The aqueous solution, method or use of embodiment 22 or embodiment 23, wherein the buffer is present at a concentration of about 0.5 mM to about 50 mM, such as about 1 mM to about 20 mM, e.g. about 2 mM to about 5 mM.

Embodiment 25. The aqueous solution, method or use of any one of embodiments 1 to 24, further comprising a non-ionic surfactant.

Embodiment 26. The aqueous solution, method or use of embodiment 25, wherein the non-ionic surfactant is an alkyl glycoside, such as dodecyl maltoside.

Embodiment 27. The aqueous solution, method or use of embodiment 25, wherein the non-ionic surfactant is a polysorbate surfactant, such as polysorbate 80 or polysorbate 20.

Embodiment 28. The aqueous solution, method or use of embodiment 25, wherein the non-ionic surfactant is an alkyl ether of polyethylene glycol.

Embodiment 29. The aqueous solution, method or use of embodiment 28, wherein the alkyl ether of polyethylene glycol is selected from polyethylene glycol (2) dodecyl ether, polyethylene glycol (2) oleyl ether and polyethylene glycol (2) hexadecyl ether.

Embodiment 30. The aqueous solution, method or use of embodiment 25, wherein the non-ionic surfactant is a block copolymer of polyethylene glycol and polypropylene glycol, such as poloxamer 188, poloxamer 407, poloxamer 171 or poloxamer 185.

Embodiment 31. The aqueous solution, method or use of embodiment 25, wherein the non-ionic surfactant is an alkylphenyl ether of polyethylene glycol, such as 4-(1,1,3,3-tetramethylbutyl)phenyl-polyethylene glycol.

Embodiment 32. The aqueous solution, method or use of any one of embodiments 25 to 31, wherein the non-ionic surfactant is present at a concentration of about 10 µg/mL to about 2000 µg/mL, such as about 50 µg/mL to about 1000 µg/mL, e.g. about 100 µg/mL to about 500 µg/mL.

Embodiment 33. The aqueous solution, method or use of any one of embodiments 1 to 32, further comprising an uncharged tonicity modifier, such as sucrose, trehalose, mannitol, sorbitol, PEG300 or PEG400.

Embodiment 34. The aqueous solution, method or use of embodiment 33, wherein the uncharged tonicity modifier is present at a concentration of about 50 mM to about 1000 mM, such as about 100 mM to about 500 mM, e.g. about 300 mM.

Embodiment 35. The aqueous solution, method or use of any one of embodiments 1 to 34, further comprising a charged tonicity modifier, such as selected from the group consisting of sodium chloride, sodium sulphate, sodium acetate, sodium lactate, glycine and histidine.

Embodiment 36. The aqueous solution, method or use of embodiment 35, wherein the charged tonicity modifier is present at a concentration of about 25 mM to about 500 mM, such as about 50 mM to about 250 mM, e.g. about 150 mM.

Embodiment 37. The aqueous solution, method or use of any one of embodiments 1 to 36, wherein the aqueous solution is isotonic.

Embodiment 38. The aqueous solution, method or use of any of embodiments 1 to 37, further comprising a preservative.

Embodiment 39. The aqueous solution, method or use of embodiment 38, wherein the preservative is selected from the group consisting of phenol, m-cresol, chlorocresol, benzyl alcohol, propylparaben, methylparaben, benzalkonium chloride and benzethonium chloride.

Embodiment 40. The aqueous solution, method or use of embodiment 38 or embodiment 39, wherein the preservative is present at a concentration of about 0.01 mM to about 100 mM.

Embodiment 41. The method of embodiment 2, or of any one of embodiments 4 to 40, wherein the method for stabilizing the antibody protein is a method for inhibiting formation of high molecular weight species of the antibody protein during storage.

Embodiment 42. The method of embodiment 2, or of any one of embodiments 4 to 40, wherein the method of stabilizing the antibody protein is a method for inhibiting formation of related species of the antibody protein during storage.

Embodiment 43. The method of embodiment 2, or of any one of embodiments 4 to 40, wherein the method of stabilizing the antibody protein is a method for inhibiting deamidation of the antibody protein during storage.

Embodiment 44. The method of embodiment 2, or of any one of embodiments 4 to 40, wherein the method of stabilizing the antibody protein is a method for inhibiting formation of low molecular weight degradation products of the antibody protein during storage.

Embodiment 45. The method of embodiment 2, or of any one of embodiments 4 to 40, wherein the method of stabilizing the antibody protein is a method for inhibiting formation of visible particles in an aqueous solution of the antibody protein during storage.

Embodiment 46. The use of any one of embodiments 3 to 40, for inhibiting formation of high molecular weight species of the antibody protein during storage.

Embodiment 47. The use of any one of embodiments 3 to 40, for inhibiting formation of related species of the antibody protein during storage.

Embodiment 48. The use of any one of embodiments 3 to 40, for inhibiting deamidation of the antibody protein during storage.

Embodiment 49. The use of any one of embodiments 3 to 40, for inhibiting formation of low molecular weight degradation products of the antibody protein during storage.

Embodiment 50. The use of any one of embodiments 3 to 40, for inhibiting formation of visible particles in an aqueous solution of the antibody protein during storage.

Embodiment 51. The aqueous solution of embodiment 1 or of any one of embodiments 4 to 40, wherein the solution is for administration by subcutaneous or intramuscular injection or by intravenous injection or infusion.

### ABBREVIATIONS

- PEG: polyethylene glycol
- HMWS: high molecular weight specie
- SEC: size exclusion chromatography
- CEX: cation-exchange chromatography

### EXAMPLES

### Materials

Arginine (Mw 174 Da), methionine (Mw 149 Da), 1,2-propanediol (Mw 76 Da), glycerol (Mw 92 Da), mannitol (Mw 182 Da), NaCl (Mw 58 Da), trehalose (Mw 342 Da) were obtained from Sigma Aldrich.

### Methods of assessing stability of an antibody protein

### (a) Visual assessment

Visible particles are suitably detected using the 2.9.20. European Pharmacepoeia Monograph (Particulate Contamination: Visible Particles). The apparatus required consists of a viewing station comprising:
- a matt black panel of appropriate size held in a vertical position
- a non-glare white panel of appropriate size held in a vertical position next to the black panel
- an adjustable lampholder fitted with a suitable, shaded, white-light source and with a suitable light diffuser (a viewing illuminator containing two 13 W fluorescent tubes, each 525 mm in length, is suitable). The intensity of illumination at the viewing point is maintained between 2000 lux and 3750 lux.

Any adherent labels are removed from the container and the outside washed and dried. The container is gently swirled or inverted, ensuring that air bubbles are not introduced, and observed for about 5 s in front of the white panel. The procedure is repeated in front of the black panel. The presence of any particles is recorded.

The visual scores are ranked as follows:
Visual score 1: Clear solution, virtually free of particles
Visual score 2: ~ 5 very small particles
Visual score 3: -10-20 very small particles
Visual score 4: 20-50 particles, including larger particles
Visual score 5: >50 particles, including larger particles

Whilst the particles in samples with visual scores 4 and 5 are clearly detectable on casual visual assessment under normal light, samples with visual score 1-3 generally appear as clear solutions on the same assessment. Samples with visual scores 1-3 are considered to be "Pass"; samples with visual score 4-5 are considered to be "Fail".

### (b) Size exclusion chromatography (SEC)

The amount of high molecular weight species is measured using a 300×7.8 mm S3000 (or equivalent) size-exclusion column with a guard column. The mobile phase is potassium phosphate pH 6.5, with a flow rate of 0.4 ml/min, injection volume of 1 µl and detected at 210 and 280 nm. The results are expressed as % high molecular species (HMWS), i.e. sum of all peak areas corresponding to aggregated protein over the sum of all protein-related peaks on the chromatogram. A small time-point to time-point variability can be observed in terms of absolute values of %HMWS, for example due to repeated size-exclusion column use. However, within a given time-point the samples are tested using the column in the same condition, so the values generated within the time-point represent a very good indication of the relative stability of the protein in the aqueous solutions tested.

### (c) Cation-exchange chromatography chromatography (CEX)

The amount of related species is measured using a Protein-Pak Hi Res SP column. Mobile phase A is 20 mM sodium phosphate (pH 6.5); mobile phase B is 20 mM sodium phosphate + 0.5 M NaCl (pH 6.0). The following gradient elution is used: 0 min - 100% A, 4 min - 80% A, 10 min - 55% A, 12 min - 0% A. Flow rate of 1.0 ml/min; injection volume is 3 µl, with UV detection at 214 nm. The results are expressed as % main peak (i.e. native protein), % acidic species and % basic species. % Related species = % acidic species + % basic species.

### Example 1

The effect of arginine, methionine and C3 polyols on the stability of rituximab (10 mg/ml) was investigated. The effect was tested in a background solution containing trisodium citrate (5 mM) and polysorbate 80 (0.7 mg/ml). All formulations tested (F1-F20) were adjusted to pH 6.5. Additional excipients in the formulations tested are shown in Table 1.

**Table 1: Additional components in formulations (F1-F20) of rituximab tested. All formulations contained rituximab (10 mg/ml), trisodium citrate (5 mM) and polysorbate 80 (0.7 mg/ml) and were adjusted to pH 6.5.**

| | Glycerol (mM) | 1,2-propanediol (mM) | Sucrose (mM) | Trehalose (mM) | NaCl (mM) | Methionine (mM) | Arginine (mM) |
|---|---|---|---|---|---|---|---|
| F1 | 150 | | | | | | |
| F2 | 150 | | | | | | 80 |
| F3 | 150 | | | | | 30 | |
| F4 | 150 | | | | | 30 | 80 |
| F5 | | 300 | | | | | |
| F6 | | 300 | | | | | 80 |
| F7 | | 300 | | | | 30 | |
| F8 | | 300 | | | | 30 | 80 |
| F9 | | | 300 | | | | |
| F10 | | | 300 | | | | 80 |
| F11 | | | 300 | | | 30 | |
| F12 | | | 300 | | | 30 | 80 |
| F13 | | | | 300 | | | |
| F14 | | | | 300 | | | 80 |
| F15 | | | | 300 | | 30 | |
| F16 | | | | 300 | | 30 | 80 |
| F17 | | | | | 300 | | |
| F18 | | | | | 300 | | 80 |
| F19 | | | | | 300 | 30 | |
| F20 | | | | | 300 | 30 | 80 |

Stability of Formulations F1-F20 was tested at 40 °C by visual assessment and size-exclusion chromatography (SEC) as described above. Results are shown in Table 2. It was shown that addition of arginine alone led to improved visual score as well as a slight reduction in the rate of HMWS formation following storage of rituximab at 40 °C in the presence of all tonicity modifiers tested (glycerol, 1,2-propanediol, sucrose, trehalose and sodium chloride). The addition of methionine alone did not appear to affect the visual score and led to a slight reduction in the rate of HMWS formation in the presence of the uncharged tonicity modifiers (glycerol, 1,2-propanediol, sucrose, trehalose), but not in the presence of sodium chloride. However, the best results (i.e. visual score 1 and the lowest rate of HMWS formation) were achieved if both arginine and methionine were added in the presence of C3 polyols. The stability of rituximab was better in these compositions (Formulations F4 and F8) than in any other compositions tested.

**Table 2: Stability of rituximab (10 mg/ml) in Formulations F1-F20 assessed by SEC and visual assessment. Formation of HMWS was assessed following storage at 40°C for 4 and 8 weeks. Visual score 1: clear solution, virtually free of particles; visual score 2: ~ 5 very small particles; visual score 3: -10-20 very small particles; visual score 4: 20-50 particles, including larger particles; visual score 5: >50 particles, including larger particles.**

| Formulation | Increase (from T0) in % HMWS (4 weeks) | Visual assessment (4 weeks) | Increase (from T0) in % HMWS (8 weeks) | Visual assessment (8 weeks) |
|---|---|---|---|---|
| F1 | 0.86 | 2 | 1.84 | 3 |
| F2 | 0.50 | 1 | 0.96 | 2 |
| F3 | 0.58 | 2 | 1.16 | 3 |
| F4 | 0.19 | 1 | 0.42 | 1 |
| F5 | 0.79 | 3 | 1.71 | 3 |
| F6 | 0.54 | 1 | 0.91 | 1 |
| F7 | 0.50 | 2 | 1.11 | 3 |
| F8 | 0.28 | 1 | 0.50 | 1 |
| F9 | 0.62 | 2 | 1.51 | 3 |
| F10 | 0.44 | 1 | 0.96 | 1 |
| F11 | 0.46 | 2 | 1.05 | 3 |
| F12 | 0.42 | 1 | 0.79 | 2 |
| F13 | 0.69 | 2 | 1.54 | 3 |
| F14 | 0.59 | 1 | 1.06 | 1 |
| F15 | 0.63 | 2 | 1.24 | 3 |
| F16 | 0.67 | 2 | 1.00 | 1 |
| F17 | 0.67 | 2 | 1.50 | 3 |
| F18 | 0.57 | 1 | 1.22 | 3 |
| F19 | 0.72 | 3 | 1.48 | 3 |
| F20 | 0.74 | 2 | 1.36 | 3 |

Throughout the specification and the claims which follow, unless the context requires otherwise, the word `comprise', and variations such as 'comprises' and 'comprising', will be understood to imply the inclusion of a stated integer, step, group of integers or group of steps but not to the exclusion of any other integer, step, group of integers or group of steps.

While this invention has been particularly shown and described with references to preferred embodiments thereof, it will be understood by those skilled in the art that various changes in form and details may be made therein without departing from the scope of the invention encompassed by the appended claims. It should also be understood that the embodiments described herein are not mutually exclusive and that features from the various embodiments may be combined in whole or in part in accordance with the invention.

All publications, patents, patent applications, internet sites, and accession numbers/database sequences (including both polynucleotide and polypeptide sequences) cited are herein incorporated by reference in their entirety for all purposes to the same extent as if each individual publication, patent, patent application, internet site, or accession number/database sequence were specifically and individually indicated to be so incorporated by reference.

## Claims

1. An aqueous solution comprising
(i) an antibody protein which is a monoclonal antibody, wherein the antibody protein is an IgG immunoglobulin; and
(ii) a stabilizing mixture of arginine, methionine and a C3 polyol selected from glycerol and 1,2-propanediol, wherein arginine is present at a concentration of 5 mM to 100 mM, methionine is present at a concentration of 2 mM to 50 mM, and the C3 polyol selected from glycerol and 1,2-propanediol is present at a concentration of 100 mM to 500 mM; and
(iii) a buffer;
wherein the pH of the solution is between about pH 5.0 and about pH 7.0.

2. The aqueous solution of claim 1, wherein the C3 polyol is 1,2-propanediol.

3. The aqueous solution of claim 1, wherein the C3 polyol is glycerol.

4. The aqueous solution of claim 1, wherein the C3 polyol is a mixture of 1,2-propanediol and glycerol.

5. The aqueous solution of any one of claims 1 to 4, wherein the monoclonal antibody is selected from trastuzumab, rituximab, bevacizumab, cetuximab and ipilimumab.

6. The aqueous solution of any one of claims 1 to 5, wherein the pH of the solution is between about pH 5.0 and about pH 6.5.

7. The aqueous solution of any one of claims 1 to 6, wherein the buffer is selected from the group consisting of citrate, histidine, succinate, maleate, acetate, phosphate and TRIS.

8. The aqueous solution of any one of claims 1 to 7, further comprising a non-ionic surfactant, wherein the non-ionic surfactant is selected from:
an alkyl glycoside, such as dodecyl maltoside;
a polysorbate surfactant, such as polysorbate 80 or polysorbate 20;
an alkyl ether of polyethylene glycol, such as polyethylene glycol (2) dodecyl ether, polyethylene glycol (2) oleyl ether and polyethylene glycol (2) hexadecyl ether;
a block copolymer of polyethylene glycol and polypropylene glycol, such as poloxamer 188, poloxamer 407, poloxamer 171 or poloxamer 185; and
an alkylphenyl ether of polyethylene glycol, such as 4-(1,1,3,3-tetramethylbutyl)phenyl-polyethylene glycol.

9. The aqueous solution of any one of claims 1 to 8, further comprising an uncharged tonicity modifier, wherein the uncharged tonicity modifier is sucrose, trehalose, mannitol, sorbitol, PEG300 or PEG400.

10. The aqueous solution of any one of claims 1 to 9, further comprising a charged tonicity modifier, wherein the charged tonicity modifier is selected from the group consisting of sodium chloride, sodium sulphate, sodium acetate, sodium lactate, glycine and histidine.

11. The aqueous solution of any of claims 1 to 10, further comprising a preservative, wherein the preservative is selected from the group consisting of phenol, m-cresol, chlorocresol, benzyl alcohol, propylparaben, methylparaben, benzalkonium chloride and benzethonium chloride.

12. The aqueous solution of any of claims 1 to 11, wherein the antibody protein is present at a concentration of about 10 mg/mL to about 200 mg/mL.

13. The aqueous solution of any of claims 1 to 12, wherein arginine is present at a concentration of 20 mM to 80 mM.

14. The aqueous solution of any of claims 1 to 13, wherein methionine is present at a concentration of 10 mM to 40 mM, for example about 30 mM.

15. The aqueous solution of any of claims 1 to 14, wherein the C3 polyol selected from glycerol and 1,2-propanediol is present at a concentration of 150 mMto 400 mM.
